# NEUE EUROPÄISCHE PATENTSCHRIFT

(11) **EP 0 988 063 B2**
(45) Veröffentlichungstag und Bekanntmachung des Hinweises auf die Entscheidung über den Einspruch: **11.08.2010**
(45) Hinweis auf die Patenterteilung: 28.08.2002
(21) Anmeldenummer: 98925314.1
(22) Anmeldetag: 10.06.1998
(51) Int. Cl.: A61L 2/00

(54) **VERFAHREN ZUR ABREICHERUNG VON VIRALEN UND MOLEKULAREN PATHOGENEN AUS EINEM BIOLOGISCHEN MATERIAL**
PROCESS FOR REDUCING THE CONCENTRATION OF VIRAL AND MOLECULAR PATHOGENS IN A BIOLOGICAL MATERIAL
PROCEDE PERMETTANT DE REDUIRE LA CONCENTRATION D'AGENTS PATHOGENES VIRAUX ET MOLECULAIRES DANS UNE MATIERE BIOLOGIQUE

(30) Priorität: 13.06.1997 AT 102997
(43) Veröffentlichungstag der Anmeldung: 29.03.2000
(60) Teilanmeldung: 00100420.9
(73) Patentinhaber: Baxter Aktiengesellschaft, 1221 Wien (AT)
(72) Erfinder: EIBL, Johann, A-1180 Wien (AT); DORNER, Friedrich, A-1230 Wien (AT); BARRETT, Noel, A-3400 Klosterneuburg (AT); PÖLSLER, Gerhard, A-1220 Wien (AT); LINNAU, Yendra, A-1220 Wien (AT)
(74) Vertreter: Alge, Daniel
(86) Internationale Anmeldenummer: PCT/AT1998/000143
(87) Internationale Veröffentlichungsnummer: WO 1998/057672

(56) Entgegenhaltungen:
- EP-A1- 0 367 840
- EP-B2- 0 131 740
- US-A- 4 590 002
- ZOLTON RAYMOND P ET AL.: "Removal of Hepatitis B Virus Infectivity from Human Gamma-Globulin Prepared by Ion-Exchange Chromatography" VOX SANGUINIS, Bd. 49, 1985, Seiten 381-389, XP002087553 in der Anmeldung erwähnt
- STANLEY M. LEMON ET AL: 'Removal/Neutralization of Hepatitis A Virus During Manufacture of High Purity, Solvent/Detergent Factor VIII Concentrate' JOURNAL OF MEDICAL VIROLOGY 1994, Seiten 44 - 49
- 'Lehrbuch der organischen Chemie', 1968, S. HIRZEL VERLAG LEIPZIG, LEIPZIG Artikel PROF. DR. HANS BEYER, Seiten 110 - 111

## Beschreibung

Die Erfindung betrifft ein Verfahren zur Abreicherung von viralen und molekularen Pathogenen aus einem biologischen Material enthaltend eine oder mehrere zu gewinnende biologische Substanzen.

Die Herstellung von therapeutischen Proteinen und Präparationen, insbesondere Immunglobulin, durch Extraktion aus humanen oder tierischen Geweben oder Flüssigkeiten, wie Blut oder Plasma, sowie aus kontinuierlich wachsenden transformierten Säugerzellen ist häufig mit dem Risiko der potentiellen Kontamination durch Pathogene, wie Viren, virus-ähnliche Partikel oder Prionen, behaftet. Es müssen daher Maßnahmen getroffen werden, daß möglicherweise vorhandene Pathogene nicht auf den Menschen übertragen werden.

Menschliches Blut bzw. Plasma kann z.B. Viren enthalten, die Krankheiten wie AIDS, Hepatitis B oder andere Hepatitis-Erkrankungen verursachen. Bei Plasmaproteinen aus Plasmapools ist das Risiko, infektiöse Agenzien wie Viren zu übertragen, aufgrund der Selektion von Blut- oder Plasmaspenden und des Herstellungsverfahrens sehr gering. Geeignete Maßnahmen, wie den Ausschluß von Blutspendern, die ein erhöhtes Risiko aufweisen, von der Blutspende sowie Analysen von Blut- oder Plasmaspenden, die es ermöglichen, infektiöse Spenden zu erfassen und von der weiteren Verteilung auszuscheiden, erlauben zwar die meisten infektiösen Spenden zu eliminieren, können aber zumeist nicht alle erfassen. Existierende Testsysteme zum Nachweis von infektiösen Viren in biologischen Materialien lassen Bedenken der potentiellen Übertragung der Pathogene nicht immer ausschließen, da es bei dem breiten Spektrum von infektiösen Pathogenen unmöglich ist, das Ausgangsmaterial auf alle Viren oder molekularen Pathogene, die in einer Probe vorhanden sein können, zu testen. Zudem erfassen die meisten Tests nicht das Virus, sondern gegen das Virus entwickelte Antikörper, so daß im Zeitraum des sogenannten "diagnostischen Fensters" kein Nachweis einer Kontamination erfolgen kann. Für einige Virusgruppen gibt es außerdem keine züverläßige oder genügend sensitive Nachweismethode. Obwohl neuentwickelte Testverfahren, insbesondere Nukleinsäureamplifikationsverfahren, wie z.B. die PCR, sehr sensitiv und spezifisch sind, können sie nur auf Pathogene angewendet werden, deren Nukleinsäuresequenz bekannt ist. In Fällen, in denen zwar die Humanpathogene bekannt sind, jedoch keine sensitiven Verfahren zum Nachweis vorhanden sind, bleibt die Unsicherheit, daß ein negatives Resultat nur aufgrund eines zu geringen Virusgehalts, der unterhalb der Sensitivitätsgrenze des Testssytems liegt, erhalten wird.

Es wurden daher für die Herstellung von pharmazeutischen und therapeutischen Produkten spezifische Entfernungs- und/oder Inaktivierungsverfahren zur Abreicherung von Viren entwickelt, so daß im Endprodukt keine infektiösen Partikel mehr zu erwarten sind.

Verschiedene Inaktivierungsverfahren basieren auf einer physikalisch-chemischen Behandlung durch Hitze und/oder Chemikalien. Als Verfahren kommen insbesondere die thermische Behandlung, das Pasteurisieren, Behandeln der Proteinlösung mit β-Propiolacton und UV-Licht, Behandeln mit einer Kombination eines Lösungsmittels und eines Detergens (sog. S/D-Verfahren) oder Belichten der Proteinlösung nach Zusatz einer photodynamischen Substanz zum Einsatz. Mit diesen Verfahren wurde eine Virusinaktivierung bis zu 10⁶ log-Stufen erreicht. Die Effizienz des Inaktivierungsverfahrens kann jedoch, abhängig vom Virustyp, variieren. Obwohl S/D-behandelte Blutprodukte als sicher in Bezug auf die Übertragung von HCV, HBV oder HIV gelten, werden nicht-umhüllte Viren, wie HAV oder Parvovirus, durch dieses Verfahren nicht inaktiviert (Prowse C., Vox Sang. 67 (1994), 191-196).

Die Hitzebehandlungsverfahren werden bei biologischen Produkten, vorzugsweise entweder in Lösung (EP-0 124 506), in trockenem Zustand (EP-0 212 040 oder WO 82/03871) oder im angefeuchteten Zustand (EP-0 324 729) durchgeführt. Oft kann es dabei zu Verlusten aufgrund der Thermolabilität vieler biologischer Substanzen kommen.

Die Art des Inaktivierungsverfahrens kann auch einen Einfluß auf die Produkte haben und eine Stabilisierung zur Minimierung des Proteinverlustes ist daher oftmals notwendig. Zudem müssen einigen Inaktivierungsverfahren Reinigungsschritte folgen, um zugesetzte Chemikalien zu entfernen.

Verfahren zur Virusabreicherung umfassen insbesondere chromatographische Verfahren, Filtration von Proteinlösungen über einen Membranfilter oder Adsorption von Viren an eine feste Phase und anschließendes Entfernen der festen Phase, wie in der EP-0 679 405 beschrieben. Es wurde jedoch festgestellt, daß die Behandlung mit einer festen Phase, wie z.B. mit Aerosil®, zwar eine Entfernung von HIV bis zu 4 log-Stufen aus einer Immunglobulin-haltigen Lösung erlaubt, der Verlust von IgG jedoch bis zu 42% betragen kann (Gao et al., Vox Sang 64 (1993), 204-209). Für den großtechnischen Ansatz ist bei diesen hohen Verlustraten ein solches Verfahren daher als eher ungeeignet anzusehen.

Ein weit verbreitetes chromatographisches Verfahren zur Isolierung von biologischen Substanzen ist die Anionenaustauscherchromatographie. Auch die Möglichkeit, daß mit diesem Trennverfahren Viren abgereichert werden können, ist in der Literatur beschrieben worden. Beispielsweise wurde die Virusabreicherung bei der Anionenaustauscherchromatographie zur Aufreinigung von vWF unter Bedingungen, unter denen der vWF, nicht aber das Virus, an den Anionenaustauscher bindet, untersucht (Burnouf-Radosevich, Vox Sang 62 (2992), 1-11). Durch gründliches Waschen der Säule vor der Elution konnte vWF - in Abhängigkeit vom Virus - mit einer Virusabreicherung von 1,5 bis 5 Zehnerpotenzen gewonnen werden.

Zolton et al. (Vox Sang 49 (1985), 381-389) untersuchten die Virusabreicherungsrate bei der Anionenaustauscherchromatographie zur Aufreinigung von gamma-Globulinen unter Bedingungen, unter denen gamma-Globuline nicht an einen Anionenaustauscher binden. Bei diesem Verfahren wurde die DEAE-Sepharose als Anionenaustauscher bei einem pH-Wert von 7,5 eingesetzt. Die Infektiösität einer mit Hepatitis B-Virus versetzten Ausgangslösung konnte dabei durch diese Anionenaustauscherchromatographie beseitigt werden. Durch dieses Experiment erfolgte eine Abreicherung des Hepatitis B-Virus um den Faktor 3000. Eine Aussage über die Abreicherungsrate anderer Viren war bei pH 7,5 jedenfalls nicht möglich. Interessanterweise traten bei einem pH-Wert von unterhalb 7,2 Viren im Durchfluß des Anionenaustauschers auf, sodaß dieses Verfahren im neutralen oder schwach sauren pH-Bereich generell als nicht anwendbar angesehen worden ist.

In der US-PS 4 590 002 wird ein Verfahren zum Entfernen von Hepatitis B-Infektivität aus gamma-Globulin enthaltenden Körperflüssigkeiten durch eine Mehrfach-Säulen-Chromatographie beschrieben.

EP 506 651 beschreibt ein mehrstufiges Verfahren zur Gewinnung einer Präparation enthaltend IgA, IgG und Transferrin, wobei während jedes einzelnen Verfahrensschritts eine Reduktion des Virustiters erreicht wurde. Während des Extraktions- und Fällungsschrittes mit 12% Ethanol konnte eine Virusreduktion um einen Faktor von 10⁵ erzielt werden. Beim Adsorptionsschritt wurden die Proteine an einen Ionenaustauscher gebunden, gewaschen und wieder eluiert. Bei diesem Schritt lag die Virusreduktion bei einem Faktor von 10³.

Burnouf (Dev. Biol. Stand. 81 (1993), 199-209) berichtet, daß durch einen Anionenaustauscherschritt bei der Aufreinigung von Faktor VIII Parainfluenzavirus und HIV-1 um 4 bzw. 3 Zehnerpotenzen abgereichert werden können. Bei der Reinigung von vWF über Anionenaustauscherchromatographie wird eine Abreicherungsrate des PRV (porcine pseudorabies virus) von 5 Log-Stufen berichtet.

Mitra et al. (Curr. Stud. Hematol. Blood Transfus. 56 (1989), 34-43) zeigen, daß bei der Aufreinigung von IgG aus Plasma nach dem Plasmafraktionierungsschema von Cohn-Oncley durch eine Folge von Fällungsschrittea in Gegenwart bestimmter Konzentrationen an Ethanol und definierter pH-Werte in der Kälte (-5°C) eine Virusabreicherung von > 5 bzw. > 8 Log-Stufen an im Maus C-Virus bzw. HIV erreicht werden kann. In dieser Arbeit wird auch berichtet, daß eine 25 %ige ethanolische Lösung bei physiologischem pH-Wert stark viruzid wirken könnte. Eine Verbindung der Ethanolbehandlung mit einer Ionenaustauscherchromatographie wird aber gemäß Mitra et al. weder gezeigt noch angeregt.

Hamman et al. (Vox Sang 67 (1994), 72-77) zeigen, daß, mittels Ionenaustauscherchromatographie, im Herstellungsprozeß eines Faktor VIII-Konzentrates eine Abreicherung der Virusaktivität bzw. Viruskonzentration von lediglich 1 bis 2 log-Stufen erreicht werden kann.

Es besteht daher ein Bedarf nach industriell anwendbaren Methoden zur sicheren Abtrennung von Viren aus Proteinlösungen, um die Infektionsrisiken für Patienten zu vermindern, die mit pharmazeutischen oder therapeutischen Präparationen tierischen, humanen Ursprungs oder hergestellt über ein gentechnisches Verfahren aus Zellkulturen, behandelt werden.

Ebenso besteht Bedarf an virussicheren, pathogenfreien biologischen Produkten, bei denen durch das Herstellungsverfahren schon sichergestellt wird, daß kein Pathogen übertragen wird und das Verfahren schonend genug ist, damit die biologische Aktivität der Produkte weitgehend unbeeinträchtigt bleibt.

Aufgabe der vorliegenden Erfindung ist daher, ein verbessertes Verfahren zur Abreicherung von viralen und molekularen Pathogenen aus einem biologischen Material zur Verfügung zu stellen.

Diese Aufgabe wird erfindungsgemäß gelöst durch ein Verfahren der eingangs geschilderten Art, welches dadurch gekennzeichnet ist, daß in einem ein-stufigen Adsorptionsverfahren ohne weitere Elution das biologische Material mit einem organischen Lösungsmittel versetzt wird, das mit dem organischen Lösungsmittel versetzte biologische Material mit einem Ionenaustauscher in Kontakt gebracht wird, wobei die Pathogene an das Ionenaustauschermaterial adsorbiert werden und zumindest eine der zu gewinnenden biologischen Substanzen keine oder nur eine geringe Wechselwirkung mit dem Ionenaustauschermaterial eingeht, und der Ionenaustauscher mit den adsorbierten Pathogenen vom biologischen Material abgetrennt wird, wobei eine virusabgereicherte Präparation der biologischen Substanz gewonnen wird.

Überraschenderweise wurde gefunden, daß in Gegenwart des organischen Lösungsmittels der Reduktionsfaktor des Ionenaustauscherschrittes im Vergleich zum im Stand der Technik beschriebenen wesentlich erhöht war. So fanden Hamman et al. (supra) einen Reduktionsfaktor von lediglich 1 log-Stufe beim Ionenaustauscherschritt.

Es war daher nicht zu erwarten, daß sich in einem ein-stufigen Verfahren, nämlich einem Adsorptionsverfahren ohne weitere Elution, ein hoher Reduktionsfaktor erreicht werden konnte. Es war ebenso überraschend, daß durch die Anwesenheit des Lösungsmittels sich die Adsorptionsspezifität des Ionenaustauschers wesentlich ändert, wodurch Pathogene gebunden werden, während biologische Substanzen, insbesondere Proteine, im wesentlichen nicht gebunden werden. So ist es mit dem erfindungsgemäßen Verfahren möglich, beispielsweise für HAV einen Reduktionsfaktor von > 5,95 log-Stufen zu erreichen, was einer vollständigen Abreicherung entspricht, während im Gegensatz dazu Hamman et al. einen Reduktionsfaktor von lediglich 1 log-Stufe für HAV mittels Ionenaustauscherschritt erzielte.

Es ist zwar bekannt, daß eine Abreicherungsbehandlung mit 12% Ethanol zur Virusreduktion beitragen kann, es war aber höchst überraschend, daß sich ein organisches Lösungsmittel auch dazu eignet, gleichzeitig mit einer Ionenaustauscherbehandlung zur Virusabreicherung angewendet zu werden.

Im Rahmen der vorliegenden Erfindung hat es sich gezeigt, daß insbesondere mit der Anionenaustauscherchromatographie bzw. der Adsorption an einem Anionenaustauscher besondere Abreicherungsraten erzielt werden können, vorzugsweise in Verbindung mit Materialien auf Basis von Kohlenhydraten oder synthetischen Polymeren, wie z.B. DEAE-Sephacel®, DEAE-Sephadex®, DEAE-Sepharose CL6B®, DEAE-Sepharose Fast Flow®, QAE-Sephadex®, Q-Sepharose Fast Flow®, Q-Sepharose High Performance®, Q-Sepharose Big Beads® (alle Fa. Pharmacia);
DEAE-Tris-Acryl®, DEAE-Spherodex®, Q-Hyper-D® (alle Fa. Sepracor) ;
Macroprep DEAE®, Macroprep Q® (alle Fa. BioRad);
DEAE-Toyopearl®, QAE-Toyopearl®, Toyopearl Super-Q® (alle Fa. Tosohaas) ;
Protein PAK DEAE® (Waters);
Fractogel EMD-TMAE®, Fractogel EMD-DEAE®, Fractogel EMD-DMAE®, Licrospher 1000 TMAE®, Licrospher 1000 DEAE® und Licrospher 4000 DMAE® (alle Fa. MERCK).
Besonders bevorzugt (vor allem bei der Immunglobulinaufarbeitung) ist ein Anionenaustauscher vom DEAE-Typ, insbesondere vom DEAE-Sephadex-Typ.

Die an den Ionenaustauscher adsorbierten Pathogene werden anschließend durch Abtrennen des Ionenaustauscher/Pathogen-Komplexes aus dem biologischen Material entfernt. Damit wird das Adsorbat unmittelbar und ohne Behandlung mit einem Elutionspuffer vom biologischen Material in einfacher Weise abgetrennt. Der Komplex wird vorzugsweise durch Penetrieren des biologischen Materials durch einen durchlässigen Filter, insbesondere einen Tiefenfilter, abgetrennt. Die Abtrennung des Komplexes kann auch durch Sedimentation, insbesondere Zentrifugation, erfolgen. Vorzugsweise erfolgt die Abtrennung des Ionenaustauschers ebenfalls in Anwesenheit des Lösungsmittels.

Mit biologischen Substanzen sind im Rahmen dieser Erfindung Substanzen biologischen Ursprungs gemeint, die beispielsweise aus Körperflüssigkeiten, wie Blut oder Plasma, gewonnen oder aus Kulturüberständen rekombinanter Zellen isoliert werden können, wobei diese biologischen Substanzen, welche im Rahmen der vorliegenden Erfindung zu gewinnen sind bzw. deren Viruskontamination abgereichert werden soll, insbesondere therapeutisch, prophylaktisch oder diagnostisch bzw. als pharmazeutische Präparate eingesetzt werden können. Bei diesen biologischen Substanzen kann es sich beispielsweise um Proteine/Peptide, Kohlehydrate oder Lipide handeln, insbesondere um biologisch aktive Substanzen, wie etwa Immunglobuline oder Blutfaktoren. Aber auch andere Stoffklassen, die von prokaryontischen oder eukaryontischen Zellen gebildet werden können, sind unter "biologischen Substanzen" zu subsummieren.

Die Gewinnung von virusabgereicherten Präparationen der biologischen Substanzen aus dem Überstand des Adsorbats bzw. aus dem Filtrat erfolgt üblicherweise durch weitere Aufbereitung oder Reinigung der biologischen Substanzen bzw. Fraktionen des biologischen Materials, wobei u.a. Fällungen, chromatographische Reinigungsverfahren, eine Filtration, Diafiltration, Formulierung vorgenommen wird.

Das biologische Material kann dabei im Rahmen der vorliegenden Erfindung eine Plasmafraktion, eine Immunglobulin-haltige Plasmafraktion, vorzugsweise COHN-Fraktion II+III, eine Plasmaprotein-haltige Fraktion enthaltend Blutfaktoren wie etwa Faktor II, Faktor VII, Faktor VIII, Faktor IX, Faktor X, Faktor XI, Protein C, Protein S, vWF, ein Konzentrat enthaltend einen der genannten Blutfaktoren, ein Überstand einer Hybridoma-Zelllinie, ein Zellkulturüberstand von transformierten oder infizierten Säugerzellen oder ein Extrakt eines tierischen oder humanen Gewebes sein. Die Parameter zur Durchführung des Verfahrens werden dabei jeweils abgestimmt auf die Art und Beschaffenheit des biologischen Materials und der möglicherweise vorhandenen kontaminierenden Pathogene. Die optimalen Parameter, wie etwa pH, Temperatur, Zeitdauer der Inkubation zur Durchführung, Art des organischen Lösungsmittels des erfindungsgemäßen Verfahrens, in Abhängigkeit von der Art des Pathogens, der Spezifität des Ionenaustauschers und der Beschaffenheit des biologischen Materials (Reinheit der Lösung, Proteinkonzentration in der Lösung), können von jedem Fachmann aufgrund seines allgemeinen Wissens ermittelt werden.

Das erfindungsgemäße Verfahren ist insbesondere dazu geeignet, molekulare und/oder virale Pathogene aus einem biologischen Material abzureichern, wobei virale Pathogene sowohl aus der Gruppe der Lipid-umhüllten, als auch aus der Gruppe der nicht-Lipidumhüllten Viren effektiv entfernt werden. Dazu zählen insbesondere Viren wie HAV, HBV, HCV, HGV, HEV, HDV, HIV, CMV oder Parvovirus.

Unter organischen Lösungsmitteln werden im Rahmen der vorliegenden Erfindung insbesondere solche Lösungsmittel verstanden, die unter den gewählten Bedingungen bei der Versetzung mit den biologischen Materialien keine wesentlichen Denaturierungsprozesse auslösen. Hiezu gehören insbesondere Lösungsmittel, wie Methanol, Ethanol oder sonstige biologisch verträgliche Alkohole.

Die optimale Konzentration des jeweiligen Lösungsmittels kann - ebenso wie geringfügige Abweichungen bei den optimalen Chromatographiebedingungen, welche gegebenenfalls durch die Anwesenheit des Lösungsmittels bedingt sein können - von jedem Fachmann durch einfache Experimente leicht ermittelt werden. Im allgemeinen wird das Lösungsmittel in einer Konzentration von 5 bis 20 Vol.%, bevorzugterweise in einer Konzentration von 10 bis 15 Vol.%, insbesondere in einer Konzentration von etwa 12 bis 14 Vol.%, angewendet.

Vorzugsweise wird als organisches Lösungsmittel ein ein- oder mehrwertiger Alkohol verwendet, wobei Ethanol besonders bevorzugt ist. Besonders bevorzugte Ethanolkonzentrationen liegen erfindungsgemäß zwischen 12 und 14 Vol.%, insbesondere zwischen 13 und 14 Vol.%.

Die Ionenaustauscherbehandlung wird vorzugsweise bei niedrigen Temperaturen durchgeführt, wobei Temperaturen unterhalb von 10°C oder unter 5°C bevorzugt werden. Wie sich gezeigt hat, sind Anionenaustauscherbehandlungen bei einer Temperatur zwischen 0 und -10°C für das erfindungsgemäße Verfahren besonders geeignet.

Es zeigte sich auch, daß das erfindungsgemäße Verfahren entgegen den Berichten des Standes der Technik, insbesondere entgegen den Ergebnissen von Zolton et al. (1985), auch bei pH-Werten unter 7,5 bzw. 7,2, also im neutralen bzw. sauren Bereich erfolgreich angewendet werden kann.

Eine bevorzugte Verfahrensvariante des erfindungsgemäßen Verfahrens liegt daher darin, daß die Behandlung des biologischen Materials mit dem Ionenaustauscher, insbesondere mit dem Anionenaustauscher, bei einem pH-Wert von 5,6 bis 7,2, bevorzugt zwischen 6,0 und 6,4, insbesondere bei pH 6,2, erfolgt.

Die Behandlung der wäßrigen Lösung des biologischen Materials mit dem Anionenaustauscher wird vorzugsweise während eines Zeitraumes von 1 Minute bis 20 Stunden, insbesondere während 4 bis 8 Stunden, vorgenommen, wobei die Inkubation entweder im Batch-Verfahren oder in einem Durchflußsystem erfolgen kann.

Mit dem erfindungsgemäßen Verfahren kann ein biologisches Material erhalten werden, das sicher frei von molekularen und/oder viralen Pathogenen ist, wobei die Pathogene im wesentlichen vollständig entfernt werden. So wurde, in Abhängigkeit der zugegebenen Ethanolkonzentration, eine im wesentlichen vollständige Virusentfernung gefunden. Durch das erfindungsgemäße einstufige Verfahren wird ein Pathogen-Reduktionsfaktor von vorzugsweise > 5,5 log-Stufen, besonders bevorzugt > 7,0 log-Stufen, erreicht

Die optimalen Adsorptionsbedingungen bei der Ionenaustauscherchromatographie können je nach zu gewinnender biologischer Substanz bzw. je nach zu adsorbierendem Virus, abhängig vom jeweiligen organischen Lösungsmittel, von jedem Fachmann leicht unter Heranziehung der Lehre der vorliegenden Erfindung optimiert werden.

Das vorliegende Verfahren eignet sich auch in hervorragender Weise zur Kombination mit weiteren Pathogen-inaktivierenden oder Pathogen-abreichernden Schritten, wie Hitze- und/oder Detergensbehandlung, Strahlenbehandlung oder Filtration, wobei die Nanofiltration gemäß der AT 403 477 B ganz besonders bevorzugt mit dem erfindungsgemäßen Verfahren kombiniert werden kann.

Das vorliegende Verfahren ist besonders geeignet zur Herstellung von Immunglobulinpräparationen, kann aber auch spezifisch zur Gewinnung von pharmazeutischen Präparaten, Blutfaktoren, wie etwa Faktor II, Faktor IIa, Faktor VII, Faktor IX, Faktor X, Protein S, Protein C oder vWF herangezogen werden.

Die vorliegende Erfindung wird durch die nachfolgenden Beispiele näher erläutert, ohne daß diese jedoch darauf beschränkt sein soll.

### Beispiele :

### Beispiel 1 : Virusabreicherung aus IgG-hältigen Lösungen (derzeit nach Ansicht der Anmelderin der beste Weg zur Ausführung der Erfindung)

Eine Immunglobulin-hältige COHN-Fraktion II+III wurde mit Ethanol bis zu einer Endkonzentration von jeweils 10% bis 14% eingestellt und die Lösung auf -3° bis -1°C temperiert. Anschließend wurde die Lösung mit den in Tabelle 1 angegebenen Testviren versetzt. Pro g Protein wurden 0,5 g DEAE-Sephadex A-50 zugesetzt, der pH bei 6,2 ± 0,1 eingestellt, und unter Konstanthaltung der Temperatur und des pH-Wertes 6 Stunden unter Rühren inkubiert. Die Austauschersuspension wurde anschließend durch Tiefenfiltration entfernt und der Virustiter im Filtrat bestimmt. Die Virusabreicherungsrate, ausgedrückt als Abnahme der infektiösen Viruspartikel in Zehnerpotenzen, ist in Tabelle 1 zusammengefaßt und wurde mittels Virustitration oder PCR bestimmt. Wie aus dieser Tabelle ersichtlich, führte dieses Verfahren - in Abhängigkeit vom verwendeten Testvirus - zu einer Abreicherung um 2 bis über 6 Zehnerpotenzen. Die Ausbeute an IgG im Überstand betrug >70%. In Kontrollversuchen, in denen die Inkubation der Testviren in analoger Weise ohne Zugabe des Anionenaustauschers durchgeführt wurde, wurde kein viruzider Effekt des Ethanols unter diesen Bedingungen festgestellt.

**Tabelle 1**

| IgG i.v. OA1-Verfahren DEAE-Sephadex und Filtration | | | | | |
|---|---|---|---|---|---|
| Ethanol | 10 % | 11 % | 12 % | 13 % | 14 % |
| Ausbeute | 100 % | 100 % | 100 % | 100 % | 97 % |

| | IRF [log] | | | | |
|---|---|---|---|---|---|
| PRV | n.d. | n.d. | 2,95 | >5,44 | >5,40 |
| MVM | 2,76 | 3,73 | >6,76 | n.d. | >6,27 |
| FSME | n.d. | n.d. | 5,00 | 5,96 | n.d. |
| BVDV | n.d. | n.d. | 1,80 | 2,61 | >4,35 |
| HCV | n.d. | >3,94 | >5,32 | n.d. | n.d. |
| HAV | n.d. | 3,91 | >5,95 | n.d. | n.d. |
| HGV | n.d. | n.d. | 4,03 | n.d. | n.d. |
| HIV | n.d. | n.d. | >4,30 | n.d. | n.d. |
| ERV | n.d. | n.d. | >5,10 | n.d. | n.d. |

| | | | | | |
|---|---|---|---|---|---|
| PRV = Pseudorabies virus MVM = Minute Virus of Mice/Parvovirus FSME = Frühsommermeningoencephalitis-Virus BVDV = Bovine diarrhea virus HCV = Hepatitis C-Virus HAV = Hepatitis A-Virus HGV = Hepatitis G-Virus HIV-1 = Human Immunodeficiency Virus-1 ERV = Equine rhinopneumonitis virus n.d. = nicht durchgeführt | | | | | |

### Beispiel 2: Abreicherung von Parvovirus aus IgG-hältigen Lösungen

Eine Immunglobulin-hältige COHN-Fraktion III mit einem Ethanolgehalt von 12% wurde in analoger Weise, wie in Beispiel 1 beschrieben, mit Parvovirus B19 versetzt. Die Gesamtbelastung im mit B19 versetzten Ausgangsmaterial betrug 1011.3 DNA-Kopien/ml. Der DEAE-Sephadex-Adsorptionsschritt mit anschließender Filtration wurde wie in Beispiel 1 beschrieben durchgeführt. Mittels PCR, wie in der AT 403 477 B beschrieben, wurden die DNA Kopien/ml im Filtrat bestimmt. Es konnte keine Parvovirus-spezifische DNA im Filtrat nachgewiesen werden. Unter Berücksichtigung der Nachweisgrenze wurde mit dem erfindungsgemäßen Verfahren ein Virus-Reduktionsfaktor von > 7,4 log-Stufen erreicht.

## Patentansprüche

1. Verfahren zur Abreicherung von viralen und molekularen Pathogenen aus einem biologischen Material, enthaltend eine oder mehrere zu gewinnende biologische Substanzen, **dadurch gekennzeichnet, daß** in einem ein-stufigen Adsorptionsverfahren ohne weitere Elution
- das biologische Material mit einem organischen Lösungsmittel versetzt wird,
- das mit Lösungsmittel versetzte biologische Material mit einem Ionenaustauscher in Kontakt gebracht wird, wobei die Pathogene an das Ionenaustauschermaterial adsorbiert werden und zumindest eine der zu gewinnenden biologischen Substanzen keine oder nur eine geringe Wechselwirkung mit dem Ionenaustauscher eingehen, und
- der Ionenaustauscher mit den adsorbierten Pathogenen vom biologischen Material abgetrennt wird, wobei eine virusabgereicherte Präparation der biologischen Substanz gewonnen wird.

2. Verfahren nach Anspruch 1, **dadurch gekennzeichnet, daß** als Ionenaustauschermaterial ein Anionenaustauscher, bevorzugt ein Anionenaustauscher vom DEAE-Typ, insbesondere DEAE-Sephadex, verwendet wird.

3. Verfahren nach Anspruch 1 oder 2, **dadurch gekennzeichnet, daß** das organische Lösungsmittel in einer Konzentration von 5 bis 20 % (v/v), vorzugsweise in einer Konzentration von 10 bis 15 % (v/v), insbesondere in einer Konzentration von 12 bis 14 % (v/v), verwendet wird.

4. Verfahren nach einem der Ansprüche 1 bis 3, **dadurch gekennzeichnet, daß** als organisches Lösungsmittel ein ein- oder mehrwertiger Alkohol, insbesondere Ethanol, eingesetzt wird.

5. Verfahren nach einem der Ansprüche 1 bis 4, **dadurch gekennzeichnet, daß** die Inkubation mit dem Ionenaustauscher bei einer Temperatur unterhalb von 10°C, vorzugsweise unter 5°C, insbesondere zwischen 0 und -10°C, erfolgt.

6. Verfahren nach einem der Ansprüche 1 bis 5, **dadurch gekennzeichnet, daß** die Inkubation mit dem Ionenaustauscher bei einem pH-Wert zwischen 5,2 und 7,2, vorzugsweise zwischen 6,0 und 6,4, insbesondere bei etwa 6,2, erfolgt.

7. Verfahren nach einem der Ansprüche 1 bis 6, **dadurch gekennzeichnet, daß** die Inkubation mit dem Ionenaustauscher 1 bis 20 Stunden, vorzugsweise 4 bis 8 Stunden, erfolgt, wobei die Inkubation entweder im Batch-Verfahren oder in einem Durchflußsystem erfolgt.

8. Verfahren nach einem der Ansprüche 1 bis 7, **dadurch gekennzeichnet, daß** als biologische Substanzen Blutfaktoren gewonnen werden.

9. Verfahren nach einem der Ansprüche 1 bis 8, **dadurch gekennzeichnet, daß** die Abtrennung des Ionenaustauschers/Pathogenen-Komplexes durch Penetrieren des biologischen Materials durch einen durchlässigen Filter erfolgt.

## Claims

1. A method for depleting viral and molecular pathogens in a biological material containing one or several biological substances to be recovered, **characterised in that** in a single-step adsorption method without further elution
- the biological material is admixed with an organic solvent,
- the solvent-admixed biological material is contacted with an ion exchanger, the pathogens being adsorbed on said ion exchanger material and at least one of the biological substances to be recovered not interacting or only slightly interacting with the ion exchanger,
and
- the ion exchanger with the adsorbed pathogens is separated from the biological material, a virus-depleted preparation of the biological substance being recovered.

2. A method according to claim 1, **characterised in that** an anion exchanger, preferably an anion exchanger of the DEAE type, in particular DEAE-Sephadex, is used as the ion exchanger material.

3. A method according to claim 1 or 2, **characterised in that** the organic solvent is used at a concentration of from 5 to 20% (v/v), preferably at a concentration of from 10 to 15% (v/v), in particular at a concentration of from 12 to 14% (v/v).

4. A method according to any one of claims 1 to 3, **characterised in that** a mono- or polyvalent alcohol, in particular ethanol, is used as the organic solvent.

5. A method according to any one of claims 1 to 4, **characterised in that** incubation with the ion exchanger is effected at a temperature of below 10°C, preferably below 5°C, in particular between 0 and -10°C.

6. A method according to any one of claims 1 to 5, **characterised in that** incubation with the ion exchanger is effected at a pH of between 5.2 and 7.2, preferably between 6.0 and 6.4, in particular at approximately 6.2.

7. A method according to any one of claims 1 to 6, **characterized in that** incubation with the ion exchanger is effected for 1 to 20 hours, preferably 4 to 8 hours, incubation being effected either in the batch method or in a continuous flow system.

8. A method according to any one of claims 1 to 7, **characterised in that** blood factors are recovered as the biological substances.

9. A method according to any one of claims 1 to 8, **characterised in that** separation of the ion exchanger/pathogen complex is effected by the biological material penetrating through a permeable filter.

## Revendications

1. Procédé permettant de réduire la concentration d'agents pathogènes viraux et moléculaires dans un matériau biologique contenant une ou plusieurs substances biologiques à extraire, **caractérisé en ce que** l'on mélange en un procédé d'adsorption en une étape sans autre (*élution*) :
- le matériau biologique avec un solvant organique,
- on met le matériau biologique mélangé avec le solvant en contact avec un échangeur d'ions, les agents pathogènes étant adsorbés sur le matériau échangeur d'ions et l'une au moins des substances biologiques à extraire ne présentant aucune interaction ou seulement une interaction minime avec l'échangeur d'ions, et
- on sépare l'échangeur d'ions avec les agents pathogènes adsorbés d'avec le matériau biologique de façon à obtenir une préparation de la substance biologique dont la concentration virale est réduite.

2. Procédé selon la revendication 1, **caractérisé en ce que** le matériau échangeur d'ions utilisé est un échangeur d'anions, de préférence un échangeur d'anions de type DEAE, en particulier de type DEAE-Sephadex.

3. Procédé selon la revendication 1 ou la revendication 2, **caractérisé en ce que** l'on utilise le solvant organique à une concentration de 5 à 20% (V/V), de préférence à une concentration de 10 à 15% (V/V), en particulier à une concentration de 12 à 14% (V/V).

4. Procédé selon l'une quelconque des revendications 1 à 3, **caractérisé en ce que** l'on utilise comme solvant organique un alcool monovalent ou plurivalent, en particulier de l'éthanol.

5. Procédé selon l'une quelconque des revendications 1 à 4, **caractérisé en ce que** l'incubation avec l'échangeur d'ions se fait à une température inférieure à 10°C, de préférence inférieure à 5°C, en particulier dans la plage de 0 à -10°C.

6. Procédé selon l'une quelconque des revendications 1 à 5, **caractérisé en ce que** l'incubation avec l'échangeur d'ions se fait à une valeur de pH dans la plage de 5,2 à 7,2, de préférence de 6,0 à 6,4, en particulier à pH 6,2 environ.

7. Procédé selon l'une quelconque des revendications 1 à 6, **caractérisé en ce que** l'incubation avec l'échangeur d'ions dure de 1 à 20 heures, de préférence de 4 à 8 heures, l'incubation se faisant soit selon un procédé par lots, soit dans un système à écoulement continu.

8. Procédé selon l'une quelconque des revendications 1 à 7, **caractérisé en ce que** les substances biologiques extraites sont des facteurs sanguins.

9. Procédé selon l'une quelconque des revendications 1 à 8, **caractérisé en ce que** la séparation du complexe échangeur d'ions/agents pathogènes se fait par la pénétration du matériau biologique à travers un filtre perméable.
